# FASCICULE DE BREVET EUROPEEN

(11) **EP 1 411 961 B1**
(45) Date de publication et mention de la délivrance du brevet: **21.09.2005**
(21) Numéro de dépôt: 02787156.5
(22) Date de dépôt: 16.07.2002
(51) Int. Cl.: A61K 35/78, A61P 21/06

(54) **UTILISATION D'EXTRAITS DE GINKGO BILOBA POUR PREPARER UN MEDICAMENT DESTINE A TRAITER LA SARCOPENIE**
VERWENDUNG VON EXTRAKTEN AUS GINKGO BILOBA ZUR HERSTELLUNG EINES MEDIKAMENTS ZUR BEHANDLUNG VON SARCOPENIA
USE OF GINKGO BILOBA EXTRACTS FOR PREPARING A MEDICINE FOR TREATING SARCOPENIA

(30) Priorité: 17.07.2001 FR 0109506
(43) Date de publication de la demande: 28.04.2004
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHRISTEN, Yves, F-75116 Paris (FR)
(74) Mandataire: Bourgouin, André
(86) Numéro de dépôt international: PCT/FR2002/002519
(87) Numéro de publication internationale: WO 2003/007973

(56) Documents cités:
- WO-A-98/00024
- US-A- 4 571 407
- US-A- 6 030 621
- DATABASE WPI Section Ch, Week 199912 Derwent Publications Ltd., London, GB; Class B04, AN 1999-136706 XP002193411 & JP 11 005746 A (YAKUKEN YG), 12 janvier 1999 (1999-01-12)
- CHANG J.Y. ET AL: "Medicinal uses of Ginkgo biloba." TODAY'S THERAPEUTIC TRENDS, (1997) 15/1 (63-74)., XP008001039
- DATABASE BIOSIS [en ligne] BIOSCIENCES INFORMATION SERVICE, PHILADELPHIA, PA, US; 1 janvier 2001 (2001-01-01) NAVARRO ANA ET AL: "Skeletal muscle and aging." Database accession no. PREV200100251287 XP002193410 & FRONTIERS IN BIOSCIENCE, vol. 6, no. Cited April 16, 2001, 1 janvier 2001 (2001-01-01), pages d26-44, Jan 1, 2001
- CHATTERJEE S.S. ET AL: "[New data in research on Ginkgo biloba]. NEUE ERGEBNISSE AUS DER GINKGO-FORSCHUNG." ARZTEZEITSCHRIFT FUR NATURHEILVERFAHREN, (1981) 22/11 (593-604). CODEN: AENADU, XP008001038
- RAPIN JEAN R ET AL: "Effects of repeated treatments with an extract of Ginkgo biloba (EGb 761) and bilobalide on liver and muscle glycogen contents in the non-insulin-dependent diabetic rat." DRUG DEVELOPMENT RESEARCH, vol. 40, no. 1, 1997, pages 68-74, XP008001026 ISSN: 0272-4391
- ALLARD M: "TREATMENT OF OLD AGE DISORDERS WITH GINKGO-BILOBA EXTRACT" PRESSE MEDICALE, vol. 15, no. 31, 1986, pages 1540-1545, XP008001037 ISSN: 0755-4982
- CHEN J X ET AL: "Ginkgo biloba extracts (EGb) induced oxidative resistance in cardiomyocytes." FASEB JOURNAL, vol. 12, no. 5, 20 mars 1998 (1998-03-20), page A1010 XP008001027 Annual Meeting of the Professional Research Scientists on Experimental Biology 98, Part II;San Francisco, California, USA; April 18-22, 1998 ISSN: 0892-6638

## Description

La présente demande de brevet concerne l'utilisation d'extraits de *Ginkgo biloba* pour préparer un médicament destiné à traiter la sarcopénie.

La sarcopénie est une affection musculaire qui touche la plupart des personnes âgées et se manifeste sous la forme d'un déclin de leur masse musculaire avec l'âge (cf. par exemple Chumlea et coll., *Nutrition, Health & Aging*, **1**(1), 7-12). Aucun remède satisfaisant n'a pour l'instant été découvert pour cette pathologie.

En effet, si l'on a par exemple montré qu'un traitement par l'hormone de croissance (GH) est capable d'accroître la masse musculaire, un tel traitement, fort coûteux, n'est pas sans présenter des effets secondaires indésirables (parmi lesquels il pourrait même y avoir un raccourcissement de la durée de vie du sujet traité).

La demanderesse a à présent trouvé que l'administration d'extraits de *Ginkgo biloba* permet de prévenir la sarcopénie ou de ralentir sa progression chez les sujets âgés qui en seraient déjà atteints.

L'invention concerne donc en premier lieu l'utilisation d'extraits de *Ginkgo biloba* pour préparer un médicament destiné à prévenir la sarcopénie ou à ralentir sa progression chez les sujets âgées qui en seraient déjà atteints.

En effet, lorsque, selon l'invention, le patient âgé est traité avec un extrait de *Ginkgo biloba*, le rapport R égal à sa masse musculaire Mₘ divisée par sa masse corporelle totale Mₜ a tendance à rester stable ou, dans la plupart des cas, à augmenter. De préférence, l'augmentation de R ainsi obtenue après une période de traitement d'un mois au moins sera supérieure ou égale à 5%, et plus préférentiellement supérieure ou égale à 6 voire 8 ou 10%.

Les extraits de *Ginkgo biloba* utilisables selon l'invention seront tels qu'ils comportent au moins des flavoneglycosides et / ou un ou des ginkgolides. De préférence, les flavoneglycosides et / ou le ou les ginkgolides seront présents au moins à hauteur de 25% en poids, plus préférentiellement au moins à hauteur de 30% en poids et encore plus préférentiellement au moins à hauteur de 50% en poids dans l'extrait de *Ginkgo biloba* utilisé pour préparer le médicament selon l'invention. Par ailleurs, la proportion de composés de type alkylphénols dans l'extrait de *Ginkgo biloba* utilisé selon l'invention sera de préférence inférieure à 10 ppm, plus préférentiellement inférieure à 5 ppm et encore plus préférentiellement inférieure à 1 ppm. Le cas échéant, le ou les ginkgolides pourront être remplacés par leurs homologues acétylés, leurs homologues alkoxylés ou leurs homologues glycosylés (comme par exemple les composés de formule générale **(I)** décrite ci-après).

De préférence, l'extrait de *Ginkgo biloba* utilisé pour préparer un médicament selon l'invention sera enrichi en flavoneglycosides et / ou en ginkgolides. Il pourra par exemple s'agir d'un extrait de type EGb 761®. Selon une autre variante de l'invention, l'extrait de *Ginkgo biloba* utilisé pour préparer un médicament selon l'invention sera tout extrait de *Ginkgo biloba* contenant des flavoneglycosides, des ginkgolides et du bilobalide, par exemple un extrait de type CP 401.

Par extrait de type EGb 761®, on entend un extrait de composition sensiblement identique à celle de l'extrait standardisé EGb 761® tel qu'il a été défini notamment dans l'article suivant : K. Drieu, La presse médicale, 31, 25 septembre 1986, supplément consacré à l'extrait de *Ginkgo biloba* (EGb 761®), 1455-1457 ; ou dans les brevets européens EP 431 535 et EP 431 536 ; par extrait de type EGb 761®, on entend donc notamment les extraits de *Ginkgo biloba* comprenant de 20 à 30 % de flavoneglycosides, de 2,5 à 4,5 % au total de ginkgolides A, B, C et J, de 2 à 4 % de bilobalide, moins de 10 % de proanthocyanidines et moins de 10 ppm (de préférence moins de 5 ppm et encore plus préférentiellement moins de 1 ppm) de composés de type alkylphénols, de préférence les extraits de *Ginkgo biloba* comprenant de 22 à 36 % de flavoneglycosides, de 2,5 à 3,5 % au total de ginkgolides A, B, C et J, de 2,5 à 3,5 % de bilobalide, moins de 8 % de proanthocyanidines et moins de 10 ppm (de préférence moins de 5 ppm et encore plus préférentiellement moins de 1 ppm) de composés de type alkylphénols, et en particulier les extraits de Ginkgo biloba comprenant environ 24 % de flavoneglycosides, 3,1 % au total de ginkgolides A, B, C et J, 2,9 % de bilobalide, 6,5 % de proanthocyanidines et moins de 1 ppm de composés de type alkylphénols.

Par extrait de type CP 401, on entend des extraits tels que ceux qui sont présentés dans le brevet US 5,389,370, notamment les extraits de *Ginkgo biloba* comprenant de 5,5 à 8 % au total de ginkgolides A, B, C et J, de 40 à 60 % de flavoneglycosides et de 5 à 7 % de bilobalide, de préférence les extraits de *Ginkgo biloba* comprenant de 6,5 à 7,5 % au total de ginkgolides A, B, C et J, de 45 à 55 % de flavoneglycosides et de 5,5 à 6,5 % de bilobalide et tout particulièrement les extraits comprenant environ 7 % au total de ginkgolides A, B, C et J, 50 % de flavoneglycosides et 6 % de bilobalide.

Par extension, seront également assimilés aux extraits de type EGb 761® ou CP 401 les extraits de type EGb 761® ou CP 401 dont les ginkgolides auront été remplacés par leurs homologues de formule générale **(I)** décrite plus loin.

Selon une variante de l'invention, au moins une partie du ou des ginkgolides pourra être remplacée par les composés de formule générale **(I)** 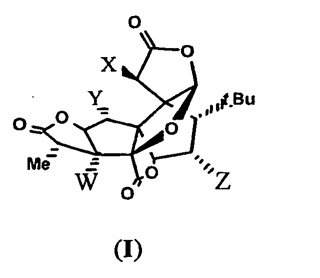 dans laquelle W, X, Y et Z représentent indépendamment les radicaux H, OH, alkoxy linéaire ou ramifié ou O-G_{S}, G_{S}-OH représentant un mono- ou un disaccharide, ou un de leurs dérivés ou analogues,
étant entendu que l'un au moins de W, X, Y ou Z représente un radical O-G_{S}.

De préférence, les composés de formule générale **(I)** décrits précédemment seront tels que X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical OH ou O-G_{S} et Z représente un radical OH ou O-G_{S} ;
- ou bien W représente un radical OH ou O-G_{S}, Y représente H et Z représente un radical OH ou O-G_{S} ;
- ou bien W représente H, Y représente un radical OH ou O-G_{S} et Z représente un radical OH ou O-G_{S} ;
- ou bien W représente un radical OH ou O-G_{S}, Y représente un radical alkoxy linéaire ou ramifié et Z représente H.

Pour la préparation des composés de formule générale **(I)**, l'homme du métier pourra se référer à la demande de brevet PCT WO 98/52959.

Les compositions pharmaceutiques comprenant un extrait de *Ginkgo biloba* peuvent être sous forme de solides, par exemple des poudres, des granules, des comprimés, des gélules, des liposomes, des suppositoires ou des timbres applicateurs (patchs). Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un extrait de *Ginkgo biloba* peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection (intramusculaire, sous-cutanée, intraveineuse, etc.), etc.

La dose d'administration journalière d'extrait de *Ginkgo biloba* envisagée est comprise entre 0,1 mg à 10 g suivant la concentration de l'extrait en principes actifs et la gravité de la sarcopénie du sujet à traiter. Il en sera en définitive décidé par le médecin ou le vétérinaire traitant.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Afin de montrer l'intérêt de l'utilisation d'extraits de *Ginkgo biloba* tels que décrits précédemment dans le traitement de la sarcopénie, le test exposé ci-après peut être effectué. D'autres tests visant par exemple à déterminer la composition corporelle et notamment le rapport entre masse graisseuse et masse non graisseuse (cf. Chumlea et coll., *Nutrition, Health & Aging,* **1**(1), 7-12) peuvent également être réalisés afin de parvenir au même résultat.

### Partie pharmacologique

### Mesure comparative de l'évolution du poids du corps et de la masse du muscle soléaire chez les rats :

### Méthode 1

3 groupes de rats sont créés : un groupe de 12 rats Wistar jeunes (4 mois) et deux groupes de rats Wistar âgés (22 mois), l'un étant constitué de 11 rats qui reçoivent une eau de boisson normale pendant 5 semaines et l'autre étant constitué de 12 rats qui reçoivent une eau de boisson contenant 75 mg par kg d'extrait de *Ginkgo biloba* standardisé EGb 761®. Après avoir été pesés, les rats Wistar jeunes sont sacrifiés à l'âge de 4 mois et 2 semaines tandis que rats Wistar âgés sont sacrifiés à l'âge de 22 mois et 3 semaines. Chez tous les groupes, les muscles Plantaris ont été pesés (muscles du côté droit) et congelés pour une analyse du typage des fibres musculaires et de modifications éventuelles des mitochondries (muscles du côté gauche).
L'effet du traitement en termes d'augmentation moyenne du poids du muscle Plantaris chez les rats traités par rapport aux rats non traités ainsi que celui en termes d'augmentation du rapport poids du muscle Plantaris sur poids total du rat est déterminée.

### Méthode 2

2 groupes de 30 à 45 rats mâles de la souche Fischer 344 sont constitués pour le test. Au début du test, les rats du premier groupe sont âgés de 12 mois et ceux du second de 24 mois.
Chacun des groupes de rats est divisé en trois sous-groupes. Les membres de chaque premier sous-groupe sont traités avec une dose journalière de 100 mg/kg d'EGb 761® administrée *per os* ; ceux de chaque second sous-groupe sont traités avec une dose journalière de 50 mg/kg d'EGb 761® administrée *per os* ; et enfin ceux de chaque troisième sous-groupe ne reçoivent aucun traitement. Tous les rats sont pesés au début du traitement et leur masses sont consignées. Après un mois de traitement et de vie dans les mêmes conditions, les rats sont pesés une dernière fois avant d'être sacrifiés par décapitation. Leurs muscles soléaires sont alors prélevés pour être pesés.
Les évolutions pondérales des masses globales des rats traités ou non par l'EGb 761® sont comparées. Les masses des muscles soléaires des rats traités ou non par l'EGb 761® sont également comparées. Ces comparaisons permettent de constater l'effet bénéfique du traitement par l'EGb 761®. En effet, ce dernier diminue la perte musculaire liée à l'âge chez le sujet traité par rapport au sujet non traité.

### Résultats obtenus dans le cas de la méthode 1 :

Les animaux traités ont gagné moins de poids par rapport aux animaux non traités (le groupe de rats âgés témoin a pris + 23% en poids en 5 semaines alors que le groupe de rats âgés traités a pris + 13,5 % en poids sur la même période).

Les mesures du poids des muscles Plantaris au terme de l'étude fournissent les résultats suivants :

| Rats jeunes | Rats âgés non traités | Rats âgés traités |
|---|---|---|
| 334,63 ± 17,82 | 273,00 ± 16,12 | 296,50 ± 21,81 |

Enfin, on constate que le poids des muscles Plantaris a augmenté de 8,60% chez les rats traités par rapport aux rats non traités alors que le rapport du poids des muscles par rapport au poids total a augmenté de 26,19% chez les rats traités par rapport aux rats non traités.

Par conséquent, on voit qu'un traitement par l'extrait de *Ginkgo biloba* standardisé EGb 761® permet bien de diminuer la perte de masse musculaire chez le sujet âgé.

## Revendications

1. Utilisation d'un extrait de *Ginkgo biloba* comportant au moins des flavoneglycosides et / ou un ou des ginkgolides pour préparer un médicament destiné à prévenir ou traiter la sarcopénie.

2. Utilisation selon la revendication 1, **caractérisée en ce que** les flavoneglycosides et / ou le ou les ginkgolides sont présents au moins à hauteur de 25% en poids dans l'extrait de *Ginkgo biloba* utilisé pour préparer le médicament.

3. Utilisation selon la revendication 1, **caractérisée en ce que** l'extrait de *Ginkgo biloba* comprend de 20 à 30 % de flavoneglycosides, de 2,5 à 4,5 % au total de ginkgolides A, B, C et J, de 2 à 4 % de bilobalide, moins de 10 % de proanthocyanidines et moins de 10 ppm de composés de type alkylphénols.

4. Utilisation selon la revendication 3, **caractérisée en ce que** l'extrait de *Ginkgo biloba* comprend moins de 5 ppm de composés de type alkylphénols.

5. Utilisation selon la revendication 3 ou 4, **caractérisée en ce qu'**au moins une partie des ginkgolides A, B, C et J est remplacée par les composés de formule générale **(I)** 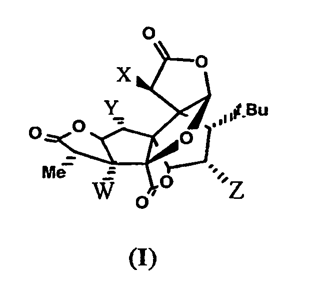 dans laquelle W, X, Y et Z représentent indépendamment les radicaux H, OH, alkoxy linéaire ou ramifié ou O-G_{S}, G_{S}-OH représentant un mono- ou un disaccharide, ou un de leurs dérivés ou analogues,
étant entendu que l'un au moins de W, X, Y ou Z représente un radical O-G_{S}.

6. Utilisation selon la revendication 5, **caractérisée en ce que** les composés de formule générale **(I)** sont tels que X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical OH ou O-G_{S} et Z représente un radical OH ou O-G_{S} ;
- ou bien W représente un radical OH ou O-G_{S}, Y représente H et Z représente un radical OH ou O-G_{S} ;
- ou bien W représente H, Y représente un radical OH ou O-G_{S} et Z représente un radical OH ou O-G_{S} ;
- ou bien W représente un radical OH ou O-G_{S}, Y représente un radical alkoxy linéaire ou ramifié et Z représente H.

7. Utilisation selon la revendication 1, **caractérisée en ce que** l'extrait de *Ginkgo biloba* comprend de 5,5 à 8 % au total de ginkgolides A, B, C et J, de 40 à 60 % de flavoneglycosides et de 5 à 7 % de bilobalide.

8. Utilisation selon la revendication 7, **caractérisée en ce que** l'extrait de *Ginkgo biloba* comprend de 6,5 à 7,5 % au total de ginkgolides A, B, C et J, de 45 à 55 % de flavoneglycosides et de 5,5 à 6,5 % de bilobalide.

9. Utilisation selon la revendication 7 ou 8, **caractérisée en ce qu'**une partie au moins des ginkgolides A, B, C et J est remplacée par les composés de formule générale **(I)** 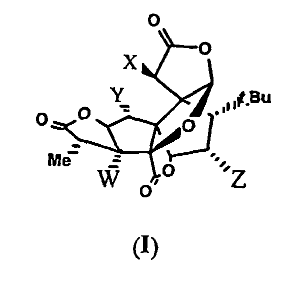 dans laquelle W, X, Y et Z représentent indépendamment les radicaux H, OH, alkoxy linéaire ou ramifié ou O-G_{S}, G_{S}-OH représentant un mono- ou un disaccharide, ou un de leurs dérivés ou analogues,
étant entendu que l'un au moins de W, X, Y ou Z représente un radical O-G_{S}.

10. Utilisation selon la revendication 9, **caractérisée en ce que** les composés de formule générale **(I)** sont tels que X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical OH ou O-G_{S} et Z représente un radical OH ou O-G_{S} ;
- ou bien W représente un radical OH ou O-G_{S}, Y représente H et Z représente un radical OH ou O-G_{S} ;
- ou bien W représente H, Y représente un radical OH ou O-G_{S} et Z représente un radical OH ou O-G_{S} ;
- ou bien W représente un radical OH ou O-G_{S}, Y représente un radical alkoxy linéaire ou ramifié et Z représente H.

## Patentansprüche

1. Verwendung eines Ginkgo-biloba-Extrakts, der mindestens Flavonglycoside und/oder ein oder mehrere Ginkgolide enthält, für die Herstellung eines Medikaments, das zur Behandlung und Prävention der Sarkopenie bestimmt ist.

2. Verwendung nach Anspruch 1, **dadurch gekennzeichnet, dass** die Flavonglycoside und/oder das oder die Ginkgolide mindestens in einer Höhe von 25 Gew.-% in dem für die Herstellung des Medikaments verwendeten *Ginkgo-biloba*-Extrakt vorliegen.

3. Verwendung nach Anspruch 1, **dadurch gekennzeichnet, dass** der *Ginkgo-biloba*-Extrakt 20 bis 30 % Flavonglycoside, insgesamt 2,5 bis 4,5 % Ginkgolide A, B, C und J, 2 bis 4 % Bilobalid, weniger als 10 % Proanthocyanidine und weniger als 10 ppm Verbindungen vom Typ der Alkylphenole umfasst.

4. Verwendung nach Anspruch 3, **dadurch gekennzeichnet, dass** der *Ginkgo-biloba*-Extrakt weniger als 5 ppm Verbindungen vom Typ der Alkylphenole umfasst.

5. Verwendung nach Anspruch 3 oder 4, **dadurch gekennzeichnet, dass** mindestens ein Teil der Ginkgolide A, B, C und J durch die Verbindungen der allgemeinen Formel (I) 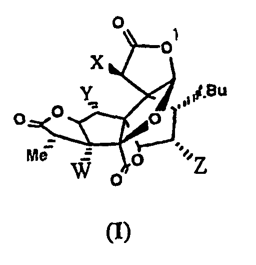 ersetzt ist, in der W, X, Y und Z unabhängig voneinander die Reste H, OH, lineares oder verzweigtes Alkoxy oder O-G_{S}, wobei G_{S}-OH ein Mono- oder ein Disaccharid darstellt, oder eines ihrer Derivate oder Analoge darstellen,
wobei gilt, dass mindestens einer der Reste W, X, Y oder Z einen O-G_{S}-Rest darstellt.

6. Verwendung nach Anspruch 5, **dadurch gekennzeichnet, dass** die Verbindungen der allgemeinen Formel (I) so gewählt sind, dass X einen OH- oder O-G_{S}-Rest, wobei G_{S}-OH ein Mono- oder ein Disaccharid darstellt, oder eines ihrer Derivate oder Analoge darstellt, und:
- W einen OH- oder O-G_{S}-Rest darstellt, Y H darstellt und Z H darstellt;
- oder W einen OH- oder O-G_{S}-Rest darstellt, Y einen OH- oder O-G_{S}-Rest darstellt und Z H darstellt;
- oder W einen OH- oder O-G_{S}-Rest darstellt, Y einen OH- oder O-G_{S}-Rest darstellt und Z einen OH- oder O-G_{S}-Rest darstellt;
- oder W einen OH- oder O-G_{S}-Rest darstellt, Y H darstellt und Z einen OH- oder O-G_{S}-Rest darstellt;
- oder W H darstellt, Y einen OH- oder O-Gₛ-Rest darstellt und Z einen OH- oder O-G_{S}-Rest darstellt;
- oder W einen OH- oder O-G_{S}-Rest darstellt, Y einen linearen oder verzweigten Alkoxyrest darstellt und Z H darstellt.

7. Verwendung nach Anspruch 1, **dadurch gekennzeichnet, dass** der *Ginkgo-biloba-*Extrakt insgesamt 5,5 bis 8 % Ginkgolide A, B, C und J, 40 bis 60 % Flavonglycoside und 5 bis 7 % Bilobalid umfasst.

8. Verwendung nach Anspruch 7, **dadurch gekennzeichnet, dass** der *Ginkgo-biloba*-Extrakt insgesamt 6,5 bis 7,5 % Ginkgolide A, B, C und *J*, 45 bis 55 % Flavonglycoside und 5,5 bis 6,5 % Bilobalid umfasst.

9. Verwendung nach Anspruch 7 oder 8, **dadurch gekennzeichnet, dass** mindestens ein Teil der Ginkgolide A, B, C und J durch die Verbindungen der allgemeinen Formel (I) 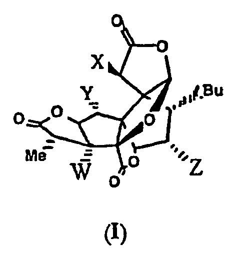 ersetzt ist, in der W, X, Y und Z unabhängig voneinander die Reste H, OH, lineares oder verzweigtes Alkoxy oder O-G_{S}, wobei G_{S}-OH ein Mono- oder ein Disaccharid darstellt, oder eines ihrer Derivate oder Analoge darstellen,
wobei gilt, dass mindestens einer der Reste W, X, Y oder Z einen O-G_{S}-Rest darstellt.

10. Verwendung nach Anspruch 9, **dadurch gekennzeichnet, dass** die Verbindungen der allgemeinen Formel (I) so gewählt sind, dass X einen OH- oder O-G_{S}-Rest, wobei G_{S}-OH ein Mono- oder ein Disaccharid darstellt, oder eines ihrer Derivate oder Analoge darstellt und:
- W einen OH- oder O-G_{S}-Rest darstellt, Y H darstellt und Z H darstellt;
- oder W einen OH- oder O-G_{S}-Rest darstellt, Y einen OH- oder O-G_{S}-Rest darstellt und Z H darstellt;
- oder W einen OH- oder O-G_{S}-Rest darstellt, Y einen OH- oder O-G_{S}-Rest darstellt und Z einen OH- oder O-G_{S}-Rest darstellt;
- oder W einen OH- oder O-G_{S}-Rest darstellt, Y H darstellt und Z einen OH- oder O-G_{S}-Rest darstellt;
- oder W H darstellt, Y einen OH- oder O-G_{S}-Rest darstellt und Z einen OH- oder O-G_{S}-Rest darstellt;
- oder W einen OH- oder O-G_{S}-Rest darstellt, Y einen linearen oder verzweigten Alkoxyrest darstellt und Z H darstellt.

## Claims

1. Use of an extract of *Ginkgo biloba* comprising at least flavone glycosides and/or one or more ginkgolides for preparing a medicament intended to prevent or treat sarcopenia.

2. Use according to claim 1, **characterized in that** the flavone glycosides and/or the ginkgolide or ginkgolides are present at least at a level of 25% by weight in the extract of *Ginkgo biloba* used for preparing the medicament.

3. Use according to claim 1, **characterized in that** the extract of *Ginkgo biloba* comprises from 20 to 30 % flavone glycosides, from 2.5 to 4.5 % in total ginkgolides A, B, C and J, from 2 to 4 % bilobalide, less than 10 % proanthocyanidines and less than 10 ppm compounds of alkylphenol type.

4. Use according to claim 3, **characterized in that** the extract of *Ginkgo biloba* comprises less than 5 ppm of compounds of alkylphenol type.

5. Use according to claim 3 or 4, **characterized in that** at least one part of the ginkgolides A, B, C and J is replaced by the compounds of general formula **(I)** 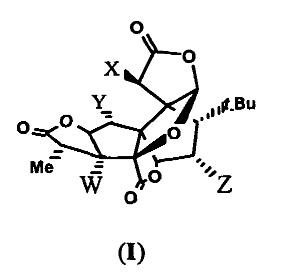 in which W, X, Y and Z independently represent the H, OH, linear or branched alkoxy or O-G_{S} radicals, G_{S}-OH representing a mono- or a disaccharide, or one of their derivatives or analogues,
it being understood that at least one of W, X, Y or Z represents an O-G_{S} radical.

6. Use according to claim 5, **characterized in that** the compounds of general formula **(I)** are such that X represents an OH or O-G_{S} radical, G_{S}-OH representing a mono- or a disaccharide, or one of their derivatives or analogues, and:
- either W represents an OH or O-G_{S} radical, Y represents H and Z represents H;
- or W represents an OH or O-G_{S} radical, Y represents an OH or O-G_{S} radical and Z represents H;
- or W represents an OH or O-G_{S} radical, Y represents an OH or O-G_{S} radical and Z represents an OH or O-G_{S} radical ;
- or W represents an OH or O-G_{S} radical, Y represents H and Z represents an OH or O-G_{S} radical ;
- or W represents H, Y represents an OH or O-G_{S} radical and Z represents an OH or O-G_{S} radical ;
- or W represents an OH or O-G_{S} radical, Y represents a linear or branched alkoxy radical and Z represents H.

7. Use according to claim 1, **characterized in that** the extract of *Ginkgo biloba* comprises from 5.5 to 8 % in total ginkgolides A, B, C and J, from 40 to 60 % flavone glycosides and from 5 to 7 % bilobalide.

8. Use according to claim 7, **characterized in that** the extract of *Ginkgo biloba* comprises from 6.5 to 7.5 % in total ginkgolides A, B, C and J, from 45 to 55 % flavone glycosides and from 5.5 to 6.5 % bilobalide.

9. Use according to claim 7 or 8, **characterized in that** at least one part of the ginkgolides A, B, C and J is replaced by the compounds of general formula **(I)** 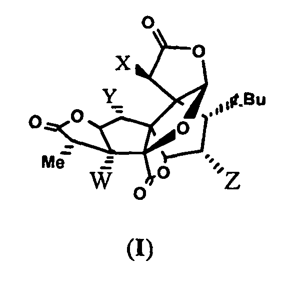 in which W, X, Y and Z independently represent the H, OH, linear or branched alkoxy or O-G_{S} radicals, G_{S}-OH representing a mono- or a disaccharide, or one of their derivatives or analogues,
it being understood that at least one of W, X, Y or Z represents an O-G_{S} radical.

10. Use according to claim 9, **characterized in that** the compounds of general formula **(I)** are such that X represents an OH or O-G_{S} radical, G_{S}-OH representing a mono- or a disaccharide, or one of their derivatives or analogues, and:
- either W represents an OH or O-G_{S} radical, Y represents H and Z represents H;
- or W represents an OH or O-G_{S} radical, Y represents an OH or O-G_{S} radical and Z represents H;
- or W represents an OH or O-G_{S} radical, Y represents an OH or O-G_{S} radical and Z represents an OH or O-G_{S} radical;
- or W represents an OH or O-G_{S} radical, Y represents H and Z represents an OH or O-G_{S} radical ;
- or W represents H, Y represents an OH or O-G_{S} radical and Z represents an OH or O-G_{S} radical ;
- or W represents an OH or O-G_{S} radical; Y represents a linear or branched alkoxy radical and Z represents H.
